# EUROPEAN PATENT APPLICATION

(11) **EP 1 357 191 A1**
(43) Date of publication of application: **29.10.2003**
(21) Application number: 02252840.0
(22) Date of filing: 23.04.2002
(51) Int. Cl.: C12N 15/82, C12N 15/29, C07K 14/415, A01H 5/00

(54) **Transgenic plants with enhanced stress tolerance**

(71) Applicant: Korea Kumho Petrochemical Co. Ltd., Jongro-gu, Seoul (KR)
(72) Inventor: Kim, Soo Young, c/o 106-401, Sangyong Apartment, Kwangju 506-302 (KR); Choi, Hyung In, 1-203, Cheil Apartment, Mokpo-Shi, Chunnam (KR)
(74) Representative: Denholm, Anna Marie

(57) **Abstract**

The present invention concerns a transgenic plant with enhanced tolerance to reduced water availability. The plant includes an expression cassette including an abscisic acid responsive element binding factor (ABF), which is capable of expressing the recombinant ABF. A method to improve plant's tolerance to reduced water availability is also provided.

SEQUENCE LISTING

<110> KOREA KUMHO PETROCHEMICAL CO., LTD.

<120> TRANSGENIC PLANTS WITH ENHANCED STRESS TOLERANCE

<160> 4

<170> KopatentIn 1.71

<210> 1
<211> 24
<212> DNA
<213> Artificial Sequence

<220>
<223> PCR primer

<400> 1
caaacttacc ctgttgttgc aact                                                24

<210> 2
<211> 34
<212> DNA
<213> Artificial Sequence

<220>
<223> PCR primer

<400> 2
ctagtctaga aggatcaagc ttctggatat ttac                                     34

<210> 3
<211> 27
<212> DNA
<213> Artificial Sequence

<220>
<223> PCR primer

<400> 3
gatcaatttg aatttttgat atacatc                                             27

<210> 4
<211> 34
<212> DNA
<213> Artificial Sequence

<220>
<223> PCR primer

<400> 4
ctagtctaga ttcaatgaaa acaaagcatc caag 34

## Description

### BACKGROUND OF THE INVENTION

The present invention relates to transgenic plants with enhanced stress tolerance. In particular, the invention relates to the transgenic plants, which express abscisic acid responsive element-binding transcription factors, ABFs, in amounts effective to confer increased tolerance to reduced water availability to the transgenic plants.

Being sessile, plants have the capability to adapt to adverse environmental conditions such as drought, cold and high salt. Under these stress conditions, the plant hormone abscisic acid (ABA) level increases in vegetative tissues, triggering adaptive responses that are essential for their survival and productivity (Leung and Giraudat, 1998; Zeevaart and Creelman, 1988). Under water deficit conditions, for example, ABA induces stomatal closure, minimizing water loss through transpiration. The ABA-controlled process is vital for plant survival, and ABA deficient or ABA response mutants are susceptible to water stress. On the other hand, high levels of ABA inhibit overall plant growth (Himmelbach et al., 1998).

Underlying the ABA-mediated stress responses is the transcriptional regulation of stress responsive gene expression (Giraudat et al., 1994; Busk and Pages, 1998). Numerous genes have been reported that are up-regulated under stress conditions in vegetative tissues (Ingram and Bartels, 1996; Shinozaki and Yamaguchi-Shinozaki, 1997). These include a class of genes known as *LEA* (Late Embryogenesis *A*bundant) genes, which are expressed abundantly in developing seeds under normal conditions, osmolyte biosynthetic genes and genes of general cellular metabolism. In general, the gene products are considered to have protective or adaptive roles under stress conditions. In addition, expression of many regulatory genes including various kinase/phosphatase and transcription factor genes is also induced by abiotic stresses. Not all stress-inducible genes are regulated by ABA. However, a large number of them are also responsive to exogenous ABA and, in many cases, their induction is impaired in ABA deficient mutants. Meanwhile, expression of some genes such as *rbcS* and *CAB* genes is suppressed by ABA and stress (Bartholomew et al., 1991; Wang et al., 1996; Weatherwax et al., 1996).

Abscisic acid responsive elements (ABREs) that control the ABA and/or stress responsive gene expression have been determined by numerous studies (Giraudat et al., 1994) and their putative cognate trans-acting factors have been isolated (Busk and Pages, 1998). Most ubiquitous among the cis-elements is a group of sequences sharing the (C/T)ACGTGGC consensus. Many of these elements contain the G-box (CACGTG) sequence (Giuliano et al., 1988), which is also present in numerous genes regulated by other environmental cues (Menkens et al., 1995). Another group of ABREs, known as "coupling element", "*hex3*" or "motif III" (Busk and Pages, 1998), shares the CGCGTG core sequence.

Based on their interactions with these two types of ABREs, which will be referred to as "ABREs" hereafter, a number of putative trans-acting factors have been isolated (Busk and Pages, 1998). Also, their homologs and numerous other G-box binding factors, all belonging to the bZIP class proteins (Landschulz et al., 1988), are able to interact with the ABREs in vitro (Foster et al., 1994). However, the evidence showing that any of these biochemically-identified factors play a role in ABA or stress signaling in planta is still lacking.

In an effort to identify transcription factors that control ABA responsive gene expression during vegetative growth, we recently isolated four ABRE binding bZIP factors by yeast one-hybrid screening of an Arabidopsis cDNA expression library (Choi et al., 2000).

Expression of the factors, referred to as ABF1 through ABF4 (*A*BRE *B*inding *F*actors *1-4*), is ABA- and stress-inducible, and they can transactivate an ABRE-containing reporter gene in yeast. ABF2 and ABF4 were reported also by Uno et al. (Uno et al., 2000), who named them as AREB1 and AREB2, respectively, and showed that the factors can activate an ABA responsive promoter in protoplasts. In order to investigate ABFs' in vivo functions, we generated transgenic Arabidopsis plants constitutively overexpressing them. Here we show that ABF3 and ABF4 transgenic lines are hypersensitive to ABA and that they exhibit several other ABA/stress-associated phenotypes including enhanced drought tolerance. ABF3 was disclosed in U.S. Pat. No. 6,218,527 (April 17, 2001), and ABF4 was disclosed in U.S. Pat. No. 6,232,461 (May 15, 2001) by the inventor himself. The expression patterns of the two ABFs correlated well with their overexpression phenotypes.

### SUMMARY OF THE INVENTION

The object of the present invention is to provide transgenic plants, which are substantially more tolerant to reduced water availability and other environmental stresses than untransformed plants, the cells of which comprise a recombinant DNA segment encoding an abscisic acid responsive element-binding factor ABF, wherein the recombinant ABF expression is in amounts effective to confer enhanced tolerance to reduced water availability and other stresses to the transformed plants.

Further, the transgenic plants have the recombinant abscisic acid responsive element-binding factor ABF3 and/or ABF4.

The present invention also provides a seed produced by the transgenic plants and a progeny, clone, cell line or cell of the transgenic plant.

### BRIEF DESCRIPTION OF THE DRAWINGS

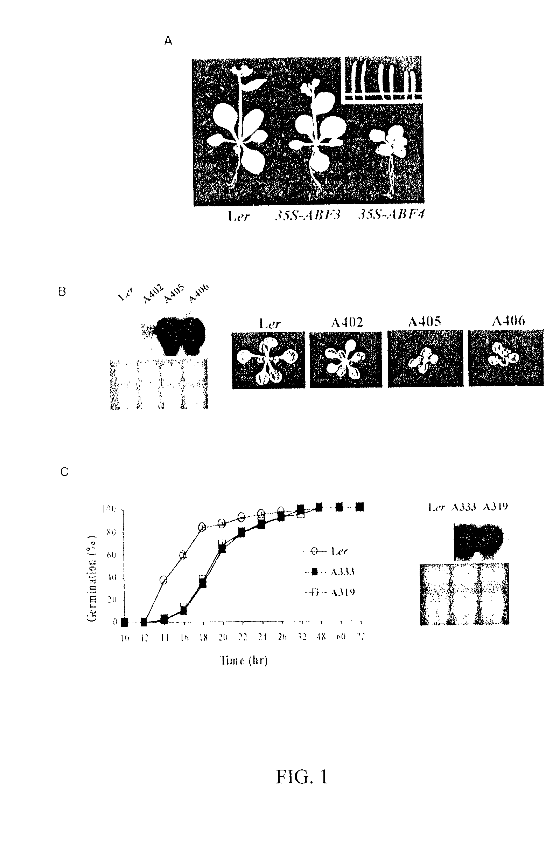

**Figure 1.** Growth Phenotype of *35S-ABF3* and *35S-ABF4* Plants. **(A)** Growth on soil. *35S-ABF3* (line A319) and *35S-ABF4* (line A405) transgenic plants were grown for 3 weeks on soil. The inset shows fully-grown siliques, two each from L*er, 35S-ABF3* and *35S-ABF4* plants (from left to right). **(B)** The relationship between the ABF4 expression level and the severity of growth phenotype. Left, RNA gel blot analysis of *ABF4* expression in L*er* and *35S-ABF4* transgenic lines, A402, A405 and A406. Lower panel shows the ethidium bromide staining of the RNA gel. Each lane contains 25 µg of total RNA. Right, *35S-ABF4* transgenic lines with varying degrees of ABF4 expression. Only the aerial parts of the plants are shown for clarity. **(C)** Left, germination of *35S-ABF3* transgenic seeds on ABA-free medium. 10 week-old seeds of L*er*, line A333 and line A319 were plated on ABA-free medium after 4 days of cold treatment, and germination (fully emerged radicle) was scored at various times. Each data point represents the means of triplicate experiments (n=50 each). Standard errors are smaller than the symbols. Right, RNA gel blot analysis of ABF3 expression in L*er* and transgenic lines A33 and A19. Lower panel shows the ethidium bromide staining of the RNA gel. Each lane contains 25 µg of total RNA. The ABF3 expression levels in A33 and A19 lines are comparable to the ABF4 expression levels in A405 and A406 lines.

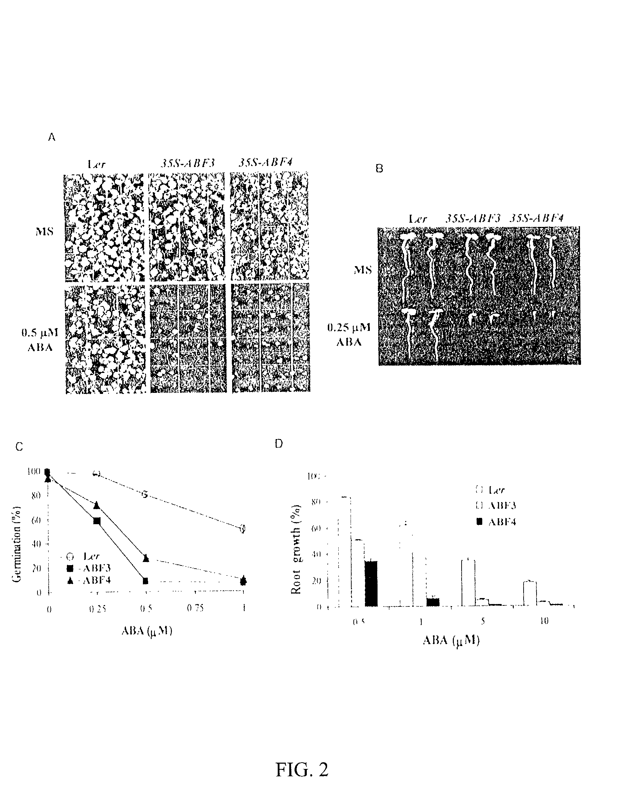

**Figure 2.** ABA Sensitivity of *35S-ABF3* and *35S-ABF4* Plants. **(A)** Growth of transgenic plants on medium containing 0.5 µM ABA. Seeds were germinated and grown for 12 days. **(B)** Growth of transgenic plants on medium containing 0.25 µM ABA. Seeds were germinated on the medium for 3 days and representative plants are shown. **(C)** ABA dose-response of germination. Seeds, 6 months old after harvest and pre-chilled at 4 °C for 4 days, were germinated on media containing various concentrations of ABA, and seedlings with fully emerged radicles were counted after 3 days. Experiments were done in triplicates (n=50 each), and the bars show standard errors. **(D)** ABA dose-response of root growth. Seeds were germinated for 4 days on ABA-free medium and the seedlings (n=6) were transferred to media containing various concentrations of ABA. Root elongation was measured 5 days after the transfer. The experiments were performed more than four times, sometimes using different transgenic lines, and the results were consistent. The small bars represent standard errors. Transgenic lines A319 (ABF3) and A405 (ABF4) were used.

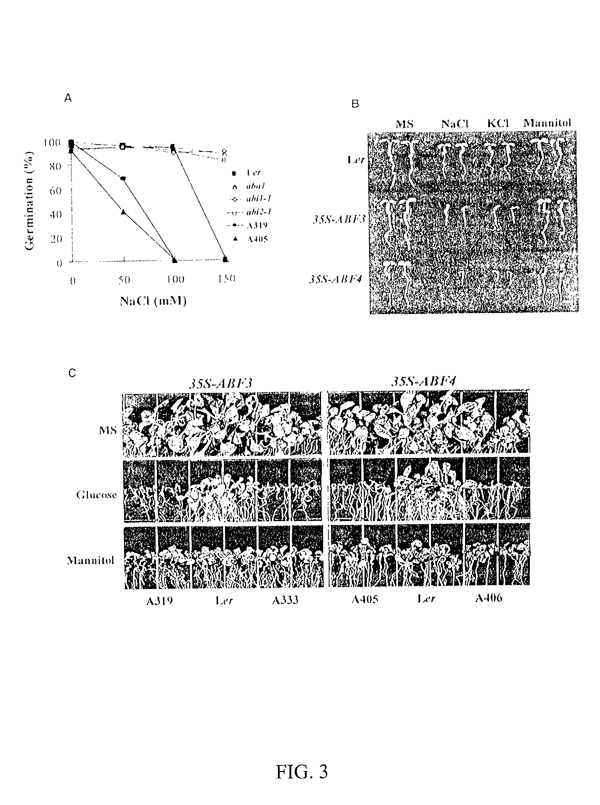

**Figure 3.** Salt and Glucose Sensitivity of *35S-ABF* Plants. **(A)** Salt effect on germination. Seeds of Ler, *aba1-1*, *abi1-1*, *abi2-1*, *35S-ABF3* (line A319) and 35S-ABP4 (line A405) were plated after 4 days of cold treatment on media containing 50, 100, or 150 mM NaCl, and germination (fully open cotyledons) was scored after 4 days. Experiments were done in triplicates (n=50 each), and standard errors are smaller than the symbols. **(B)** Salt effect on newly germinated seedling growth. Seeds of the same transgenic lines were germinated for 4 days on media containing 100 mM of NaCl , KCl or mannitol, and representative seedlings are shown. **(C)** Glucose response of *35S-ABF* transgenic plants. Seeds were germinated and grown for 14 days on the regular growth medium or the same medium supplemented with 3% glucose or mannitol at vertical position. A319, A333, A405, and A406 indicate transgenic lines.

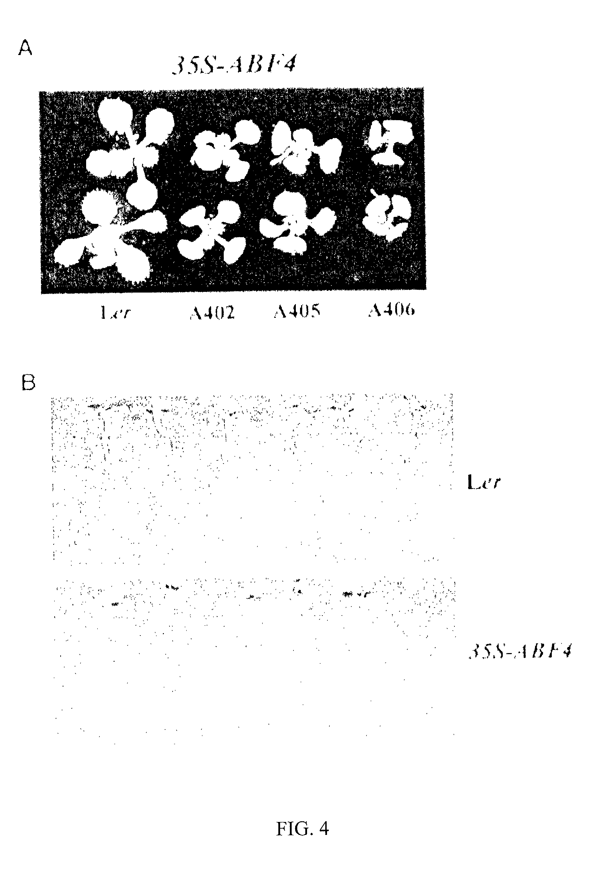

**Figure 4.** Epinasty and Obstacle-touching Response of *35S-ABF4* Plants. **(A)** Epinastic curvature of *35S-ABF4* transgenic leaves. L*er* and transgenic plants were grown on MS plates for 2 weeks. *ABF4* expression level is highest in the A406 line and lowest in the A402 line (Figure 1B). **(B)** Obstacle-touching response of *35S-ABF4* plants. Plants (line A405) were grown on 1.5% agar plates for 4 days at vertical position and then for 5 days at an angled (45°) position. The arrows indicate the root tip position before changing to the slanted orientation.

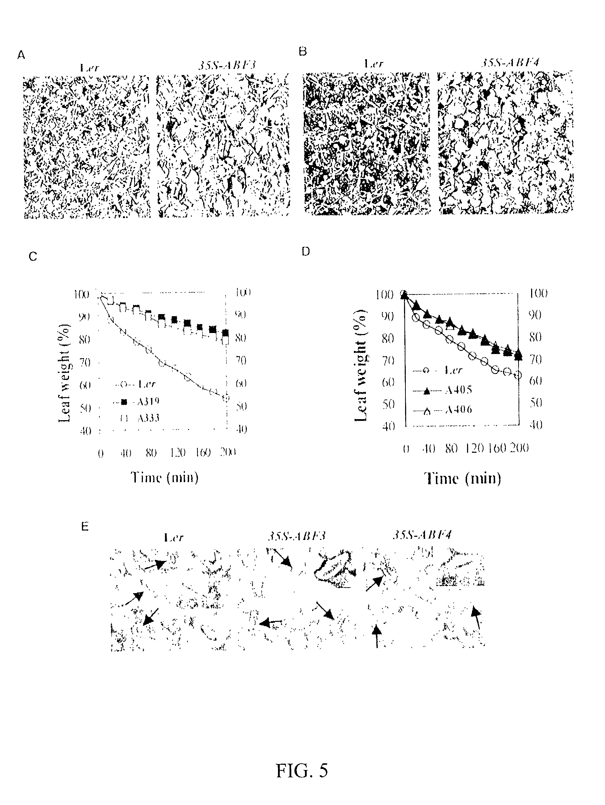

**Figure 5.** Drought Tolerance of *35S-ABF3* and *35S-ABF4* Plants. **(A)** Drought tolerance of *35S-ABF3* transgenic plants (line A319). Transgenic and wild type plants (n=100 each) were grown on soil in the same container for two weeks, withheld from water for 11 days, and then re-watered. The picture was taken 3 days after the rewatering. **(B)** Drought tolerance of *35S-ABF4* transgenic plants (line A405). Plants at similar developmental stages (two weeks old wild type plants and 3 weeks old ABF4 plants) were withheld from water for 12 days and then re-watered. The picture was taken 3 days after the re-watering. **(C)** and **(D)** show the transpiration rates of *35S-ABF3* and *35S-ABF4* transgenic plants, respectively. Leaves of similar developmental stages were excised and weighed at various times after the detachment. Each data point represents the mean of duplicate measurements (n=9 each). Standard errors are smaller than the symbols. **(E)** Stomatal aperture of ABF transgenic plants (lines A319 and A405). Stomatal guard cells were observed in the middle of watering period. Arrows indicate guard cells, and the insets show representative stomata.

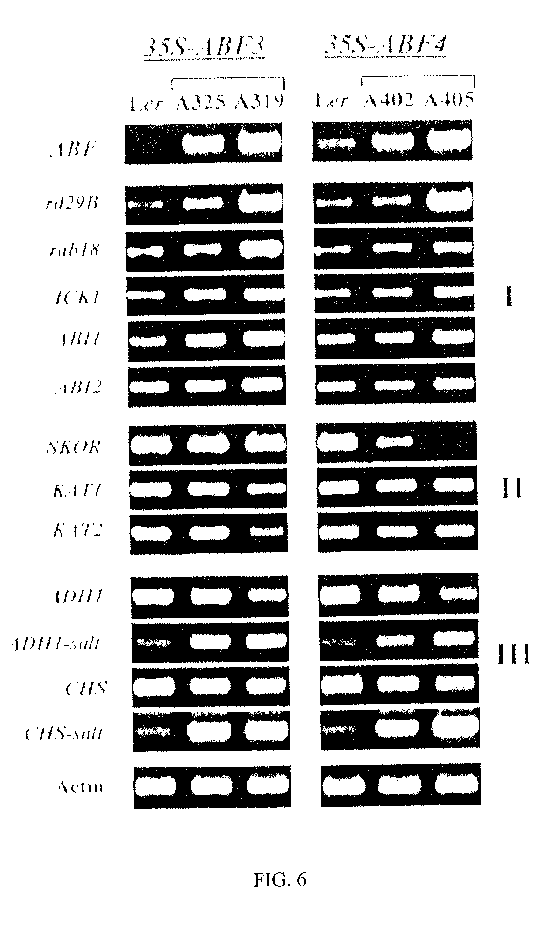

**Figure 6.** Expression of ABA-regulated Genes in *35S-ABF* Transgenic Lines. RNA levels of ABA responsive genes were determined by RT-PCR, using total RNAs isolated from 2 week old plants grown on MS plates. Lines A319 and A405 represent transgenic lines with higher *ABF* expression, whereas lines A325 and A402 represents transgenic lines with lower *ABF* expression.

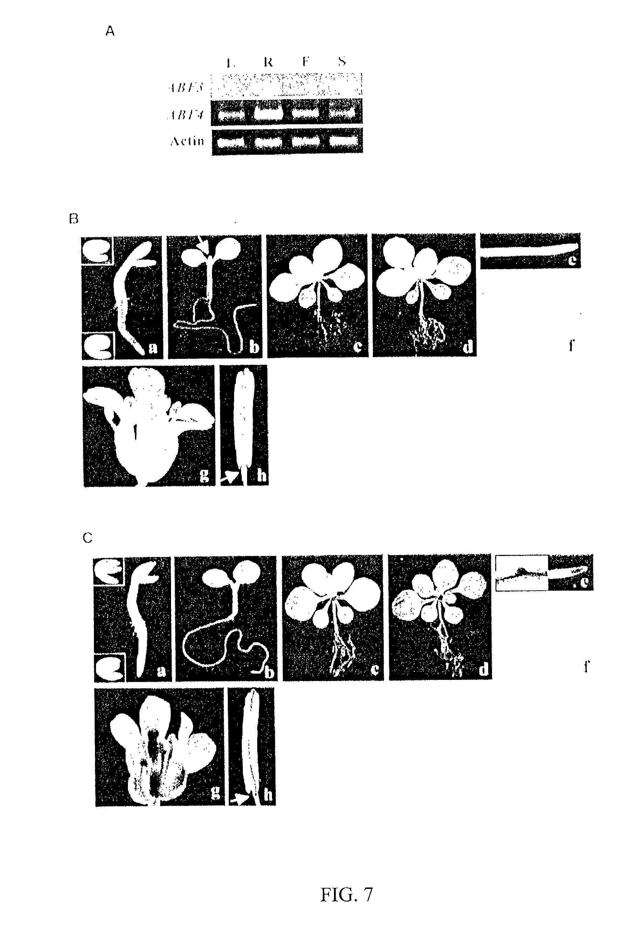

**Figure 7.** Expression Patterns of *ABF3* and *ABF4*. **(A)** Tissue-specificity of *ABF3* and *ABF4* expression. RNA was isolated from various tissues of wild type plants grown under normal condition and the expression of *ABF3* and *ABF4* was determined by RT-PCR. L, leaves from three-week-old plants. R, roots from three-week-old plants. F, flowers. Si, immature siliques. **(B)** and **(C)** Histochemical GUS staining of the ABF3 and the ABF4 promoter activity, respectively. T3 homozygous plants were stained with X-gluc for 6 (panels e and f) or 24 hrs (other panels). a, 2-day-old seedlings. Insets show embryos from immature siliques (top) or dry seeds (bottom). b. 5 day-old seedlings. The arrow in **(B)** shows the newly emerging shoot. c. 2-week-old seedlings, d. 2-week-old seedlings treated with 100 µM ABA (ABF3) or 200 mM NaCl (ABF4). e, root tips. The left half of the panel in **(C)** shows a lateral root primordium. f, guard cells. g, flowers. h, siliques. Arrows indicate the silique abscission zone.

### DETAILED DESCRIPTION OF THE INVENTION

### Growth Phenotypes of ABF3 and ABF4 Overexpression Lines

In order to investigate the in vivo functions of ABF3 and ABF4, we employed an overexpression approach. The coding region of *ABF3* or *ABF4* was fused to the cauliflower mosaic virus *35S* promoter and each construct was used to transform Arabidopsis (ecotype L*er)* plants. Thirtyeight and twelve T3 homozygous lines were recovered from the *35S-ABF3* and the *35S-ABF4* constructs, respectively, and, after preliminary analysis, transgenic lines with higher ABF expression levels were selected for more detailed analysis.

Compared with wild type plants, *35S-ABF3* transgenic plants exhibited mild growth retardation in the aerial parts; petioles were slightly shorter and leaves were rounder in shape (Figure 1A). The degree of retardation was not severe, however, and overall growth patterns were similar to wild type plants except that siliques were somewhat shorter and thicker (Figure 1A, inset). In contrast, *35S-ABF4* transgenic plants exhibited severe growth retardation (Figure 1A), which was dependent on the *ABF4* expression level (Figure 1B). Petioles were shorter, leaves were smaller, flowering was delayed, and plants were shorter. Also, germination of *35S-ABF3* plants was delayed several hours compared with that of wild type plants in the absence of ABA (Figure 1C). *35S-ABF4* plants, on the other hand, germinated normally (data not shown), and, thus, the growth retardation observed with *35S-ABF4* plants was a post-germination process.

### ABA Response of 35S-ABF Plants

To test whether ABF3 or ABF4 overexpression affected ABA sensitivity, *35S-ABF3* and *35S-ABF4* transgenic plants were germinated and grown on media containing various concentrations of ABA. When ABA concentration was 0.5 µM or above, the growth of *35S-ABF* plants was completely arrested after radicles emerged, i.e., cotyledon greening/expansion and root growth were severely inhibited and none of the transgenic seedlings developed to have true leaves (Figure 2A). At the same conditions, wild type plants continued to grow and develop although at slower rates than on ABA-free medium. The ABA hypersensitivity of *35S-ABF* transgenic plants was also observed at 0.25 µM ABA (Figure 2B), although the transgenic seedlings eventually grew to have true leaves (data not shown).

To see the stage-specificity of ABA response, ABA dose-response was examined during and after germination. As shown in Figure 2C, *35S-ABF* transgenic plants were hypersensitive to ABA at the germination stage, 0.5 µM ABA being sufficient to inhibit their germination efficiencies to 8 (ABF3) or 28% (ABF4) while wild type plants retained 80% germination at the same condition. Likewise, root growth of the *35S-ABF* transgenic lines was hypersensitive to ABA (Figure 2D). At 0.5 µM ABA, wild type root growth was 84% of its control rate, whereas those of *35S-ABF4* and *35S-ABF3* plants were 35% and 51% of their control rates, respectively. At 1 µM ABA, root growth of the *35S-ABF4* plants reduced to 6% and the *35S-ABF3* plants to 37%. In addition, lateral root and aerial part growth of *35S-ABF* transgenic plants was significantly inhibited at this concentration (data not shown). Wild type plants grew at 62% of the control rate at the same ABA concentration. Transgenic root growth was almost completely arrested when ABA concentration was above 5 µM, whereas wild type plants still continued to grow. The results indicate that both germination and post-germination growth of *35S-ABF* transgenic plants are hypersensitive to ABA.

### Salt Response of 35S-ABF Plants

High concentrations of salts inhibit the germination of Arabidopsis (Werner and Finkelstein, 1995; Leon-Kloosterziel et al., 1996; Quesada et al., 2000; Zhu, 2000). Several studies show that ABA plays a role in the inhibition process. Although not all salt-insensitive mutants are ABA-insensitive (Warner and Finkelstein, 1995), all ABA deficient (*aba*) and ABA insensitive (*abi*) mutants exhibit salt-insensitivity during germination (Leon-Kloosterziel et al., 1996). This is probably because ABA, whose level increases by high salt, promotes the inhibition process. Since the expression of both ABF3 and ABF4 is salt-inducible (Choi et al., 2000), they may participate in salt response, i.e., salt-induced germination inhibition in this case. To test this, *35S-ABF* transgenic plants were germinated on media containing various concentrations of NaCl. Figure 3A shows that the germination of wild type and *aba1, abi1* and *abi2* mutant plants was not affected by NaCl below 100 mM. In contrast, germination and growth of the *35S-ABF* plants were significantly affected by 100 mM NaCl (Figure 3, A and B); radicle emergence, root growth and cotyledon opening/expansion were severely inhibited. In a parallel experiment, the transgenic plants responded to KCl in a similar way to NaCl (Figure 3B). On the other hand, their response to the same concentration or twice the concentration (data not shown) of mannitol, which gives the same osmotic pressure, was normal. Thus, in contrast to the salt-insensitive phenotype of ABA deficient or ABA insensitive mutants, ABF3 and ABF4 overexpression both resulted in salt hypersensitivity at the germination/young seedling stage, and the hypersensitivity appeared to be ionic rather than osmotic in nature.

### Sugar Response of 35S-ABF Plants

At higher concentrations, sugars inhibit the development of young seedlings, i.e., they inhibit cotyledon greening/expansion and shoot growth (Jang et al., 1997). According to works done by others, ABA plays an essential role in the glucose or sucrose signal transduction (Arenas-Huertero et al., 2000; Huijser et al., 2000; Laby et al., 2000). For example, the ABA-deficient, *aba2* mutation is allelic to the sugar insensitive, *sis4* mutation, and the glucose or sugar insensitive mutations, *gin6*, *sis5 and sun6* are allelic to the ABA insensitive mutation *abi4*. Also, the studies show that other *aba* mutants and, to some degree, *abi5* mutants are insensitive to glucose. Thus, ABF overexpression might have affected sugar sensitivity as well, if indeed ABF3 and ABF4 mediate ABA signaling. We addressed this by examining their response to glucose, which exerts more severe growth inhibition than other sugars (Jang et al., 1997). Under our experimental conditions, wild type seedlings showed growth defects such as the inhibition of cotyledon greening and true leaf development when glucose concentration was above 4% (data not shown). The aerial part growth of *35S-ABF* transgenic lines, on the other hand, was arrested completely at 3% glucose, at which wild type plants developed fully (Figure 3C). Thus, *35S-ABF* transgenic plants were hypersensitive to glucose. This enhanced response of the transgenic plants was not observed with the same concentration of mannitol, which inhibited the growth of both wild type and *35S-ABF* transgenic plants significantly but similarly. Thus, the hypersensitivity was glucose-specific rather than osmotic.

### Epinasty and Obstacle-touching Response of 35S-ABF4 Plants

Recent genetic studies show that ABA signaling pathways interact with those of ethylene. According to the studies, ABA-mediated inhibition of germination is negatively regulated by ethylene, whereas ethylene signaling components are required for the ABA inhibition of root growth (Beaudoin et al., 2000; Ghassemian et al., 2000). The involvement of auxin in ABA-dependent stress response has also been demonstrated (Vartanian et al., 1994). When exposed to progressive drought, new lateral roots take a short and tuberized form. The process, known as 'drought rhizogenesis', is impaired not only in the ABA deficient *aba-1* and ABA-insensitive *abi1-1* mutants but also in the auxin resistant *axr1-3* mutant. To test whether the overexpression of ABF3 or ABF4 affected ethylene or auxin sensitivity, we compared the effects of the ethylene precursor 1-aminocyclopropane-1-carboxylic acid (ACC) and indole-3-acetic acid (IAA) on the growth of *35S-ABF* and wild type plants. We did not see any differences in their responses. However, leaves of 35S-ABF4 plants have a tendency to have epinastic curvature when grown on plates (Figure 4A), which is generally attributed to ethylene action under stress conditions (Jackson, 1997). Also, roots of *35S-ABF4* transgenic plants exhibited abnormality in the obstacle-touching response (Okada and Shimura, 1990; Simmons et al., 1995). As shown in Figure 4B, roots of wild type plants grow in a wavy pattern when grown on a hard agar surface at an inclined position. The wavy pattern of root growth, however, was significantly diminished in *35S-ABF4* plants. The obstacle-touching response is impaired in several auxin resistant mutants, indicating that auxin signalling components are involved in the process (Okada and Shimura, 1992). Thus, the result implies that auxin signaling pathway(s) might have been perturbed in *35S-ABF4* plants. Taken together, our results suggest that ABF4 overexpression might have affected some aspects of ethylene and auxin responses.

### Drought Tolerance of 35S-ABF Plants

One of the key ABA-controlled processes is the stomatal closure under water stress conditions, which minimizes water loss through transpiration (Leung and Giraudat, 1998). ABA biosynthesis mutants and some of the ABA response mutants (i.e., *abi1* and *abi2*), therefore, are very susceptible to drought because of the impaired stomatal aperture regulation (Leung and Giraudat, 1998; Schroeder et al., 2001). Thus, ABF3 and ABF4 overexpression lines are expected to exhibit altered response to water deficit conditions if the factors are involved in ABA/stress signaling. To address this, we examined the drought tolerance of *35S-ABF* plants. As shown in Figure 5A, wild type plants withered completely when withdrawn from water for 11 days and only 16% of them survived to maturity when re-watered afterwards. *35S-ABF3* plants, however, were not affected noticeably and all survived the treatment to set seeds. In a similar experiment, *35S-ABF4* plants also exhibited higher survival rates under water deficit condition; all of them survived a 12 day drought treatment, whereas 33% of the wild type plants survived to set seeds (Figure 5B). Thus, both *35S-ABF3* and *35S-ABF4* plants survived the drought conditions better than wild type plants.

The enhanced drought tolerance of the transgenic plants could be attributed, at least in part, to their lower transpiration rates. When measured by the fresh weight loss of detached rosette leaves, the water loss rates of *35S-ABF3* and *35S-ABF4* transgenic lines were less than half and approximately 70% of the wild type plants', respectively (Figure 5, C and D). Consistent with the result, the stomata of the transgenic plants had smaller openings than wild type plants (Figure 5E). Under normal growth condition, ca. 85% (89/104) of the wild type stomata were open, whereas approximately 20% of them were open in *35S-ABF3* (15/71) and *35S-ABF4* (17/71) plants when observed in the middle of watering period. Thus, constitutive overexpression of ABF3 or ABF4 resulted in partial stomatal closure, reduced transpiration and enhanced drought tolerance.

### Expression of ABA Responsive Genes in 35S-ABF Plants

To investigate the transcriptional regulatory roles of ABF3 and ABF4 in planta, expression of various ABA/stress responsive genes in *35S-ABF* plants was determined. As shown in Figure 6, the transcript levels of a number of ABA-regulated genes (Group I) were enhanced in *35S-ABF3* and *35S-ABF4* transgenic lines. These include *LEA* class genes *rd29B* (Yamaguchi-Shinozaki and Shinozaki, 1994) and *rab18* (Lang and Palva, 1992), whose expression is induced by ABA and abiotic stresses. Expression of the ABA-inducible, cell cycle regulator gene *ICK1* (cyclin-dependent kinase inhibitor) (Wang et al., 1998) was also elevated in the *35S-ABF* transgenic lines. The *ICK1* gene has been suggested to mediate cell division arrest by ABA. In the *35S-ABF3* lines, strong enhancement of *ABI1* (Leung et al, 1994; Meyer et al., 1994) RNA level was observed, and *ABI2* (Leung et al., 1997) transcript level was elevated albeit the degree of increase was lower. Increase in the *ABI1* RNA level was also observed in the *35S-ABF4* line with higher *ABF4* expression (A405). Expression of *ABI1* and *ABI2*, which encode homologous protein phosphatase 2Cs and whose mutations result in defective stomatal closing and wilty phenotype (Schroeder et al., 2001), is enhanced by ABA and water stress (Leung et al., 1997). Meanwhile, the RNA level of the ABA-repressible gene *SKOR* (Gaymard et al., 1998) was significantly reduced or undetectable in *35S-ABF4* transgenic lines (Group II). The gene encodes a root-specific K⁺ outward rectifying channel, and ABA repression of its expression has been suggested to be a part of adaptive water stress response. Similarly, guard cell ion channel genes *KAT1* and *KAT2* (Anderson et al., 1992; Pilot et al., 2001) were negatively regulated in the *35S-ABF3* line with higher *ABF3* level (A319). The two ion channels normally mediate K⁺ influx, enabling stomatal opening, but their activity is inhibited by ABA. Also, stress responsive biosynthetic genes (Group III), chalcone synthase gene *CHS* (Feinbaum and Ausubel, 1988) and alcohol dehydrogenase gene *ADH1* (de Bruxelles et al., 1996), were down-regulated. The transcript levels of the two genes were, however, higher in the *35S-ABF* transgenic lines when plants were treated with high salt, suggesting that stress-induced post-translational modification of ABF3 and ABF4 may be required for the regulation of these genes. In summary, overexpression of ABF3 or ABF4 resulted in the modulation of ABA/stress responsive gene expression and the two factors played positive or negative roles depending on specific genes and environmental conditions.

### Expression Patterns of ABF3 and ABF4

The phenotypes described so far indicated that overexpression of ABF3 or ABF4 enhanced various aspects of ABA response. To assess the physiological relevance of the results and to obtain further clues about their functions, we investigated their temporal and spatial expression patterns. We first investigated the tissue-specificity of their uninduced, basal expression. Since the basal expression levels of ABF3 and ABF4 are very low (Choi et al., 2000), we employed the coupled reverse transcription and polymerase chain reaction (RT-PCR) for the analysis. As shown in Figure 7A, relatively higher expression of both *ABF3* and *ABF4* was detected in roots. Also, lower ABF3 expression was observed in flowers but not in leaves and siliques at the same condition. On the other hand, weak *ABF4* expression was detectable in leaves, flowers and siliques.

More detailed temporal and spatial expression patterns of *ABF3* and *ABF4* were determined by histochemical GUS staining of transgenic plants that harboured an *ABF promoter-GUS* reporter construct. With the *ABF3* promoter (2.1 kb) construct, GUS activity was undetectable in embryos, but it was observed in the emerging radicles at the germination stage (Figure 7B, a) and in most of the vegetative tissues at later stages (Figure 7B, b and c). Roots were stained most strongly except the tip area, and petioles, leaf vascular tissues and guard cells exhibited relatively strong GUS activity (Figure 7B, b-f). Emerging shoots and younger leaves (Figure 7B, b and c), on the other hand, exhibited GUS staining only after ABA, salt or mannitol treatment (Figure 7B, d). In mature plants, GUS staining was detected also in anthers, stigma, and siliques (abscission zone, replum, and funiculi) (Figure 7B, g and h).

The *ABF4* promoter was active in embryos from green siliques (Figure 7C, a), but its activity decreased as embryos became mature and was not detected in newly germinated seedlings except in some limited regions (radicle tip, shoot meristem region, and cotyledon tips) (Figure 7C, a). At later stages, starting from the stage of fully-expanded cotyledons (Figure 7C, b), *ABF4* promoter activity was observed in all vegetative tissues (Figure 7C, c-f), and also in floral organs and siliques (abscission zone, replum, and funiculi) (Figure 7C, g and h). The *ABF4* promoter was most active in roots, especially in the growing regions (meristem, elongation zone and lateral root primordia) (Figure 7C, e), suggesting its role in growth regulation. Also, it exhibited strong activity in petioles and guard cells (Figure 7C, c and f). Salt treatment of seedlings enhanced the *ABF4* promoter activity somewhat (Figure 7C, d).

### DISCUSSION

ABA-regulated gene expression plays a central role in ABA signaling, and numerous ABA/stress responsive genes are regulated by the (C/T)ACGTGGC- or CGCGTG-containing. ABREs. Thus, identifying relevant transcription factors is critical for the delineation of ABA signal transduction cascades. Many studies show that ABA signaling pathways are tissue-specific (Leung and Giraudat, 1998; Giraudat et al., 1994), and several seed-specific ABA signaling components (*ABI3, ABI4*, and *ABI5*) have been identified by genetic screens. *ABI3* and *ABI4* encode transcription factors (Giraudat et al., 1992; Finkelstein et al., 1998), whose binding sites and immediate target genes are unknown. Recently, *ABI5* has been shown to encode a bZIP factor that belongs to a seed-specific subfamily of ABF-related factors (Finkelstein and Lynch, 2000; Lopez-Molina and Chua, 2000) and its role in postgermination developmental arrest has also been demonstrated (Lopez-Molina et al., 2001). However, ABRE binding factors whose major function is to mediate the ABA signaling during vegetative growth have not been reported, although numerous bZIP factors are known to interact with the ABREs in vitro (Foster et al., 1994).

ABFs are unique among the ABRE binding bZIP factors in that, unlike most of the other plant bZIP factors, they can interact with both the G-box type and the CGCGTG-containing ABREs (Choi et al., 2000). The broad binding specificity, together with their transactivation capability of an ABRE-containing reporter gene and the stress-inducibility of their expression, suggested that ABFs have a potential to regulate a large number of ABA/stress responsive genes and, thus, are likely to participate in stress responsive ABA signaling. In order to address this question, we employed an overexpression approach. Considering the potential functional redundancy of ABFs and numerous other bZIP factors interacting with ABREs (Foster et al., 1994), the approach would be a better way than loss of function approaches such as antisense, knockout and RNA interference. The overexpression of ABFs (we estimate that *ABF3* and *ABF4* levels in the *35S-ABF* transgenic lines used in our study range from approximately 2 to 10 folds of their ABA-induced levels), however, might have caused non-natural conditions. For example, genes that are not normally regulated by ABFs might have been turned on or off. Also, it may have affected the functions of other members of ABFs or potentially other bZIP factors, by titrating them out via non-natural heterdimerization. Thus, the overexpression phenotypes need to be interpreted with caution, and their roles can be further confirmed by other experimental means. Nevertheless, our results show that ABF3 or ABF4 overexpression conferred several ABA-associated phenotypes such as ABA hypersensitivity, sugar hypersensitivity and enhanced drought tolerance, with altered expression of ABA/stress responsive genes. Thus, our data provide a strong in vivo case for the involvement of ABF3 and ABF4 in stress responsive ABA signaling.

Whereas the ABA hypersensitivity conferred by ABF3 or ABF4 overexpression was very distinct and observed both at the germination and at later growth stages (Figure 2), the overexpression effects on growth in the absence of exogenous ABA were either moderate (ABF3) or developmental stage-dependent (ABF4) (Figure 1). ABF3 exerted inhibitory effect on both germination and seedling growth. However, the low degree of inhibition compared with that in the presence of exogenous ABA suggests that ABF3 alone is not sufficient for the inhibitory function. On the other hand, ABF4 overexpression had little effect on germination but had a severe effect on seedling growth, suggesting that ABF4 activity is developmentally modulated. Alternatively, the result may indicate that ABA inhibition of seedling growth is mediated by a mechanism that differs from the germination inhibition mechanism. Also, the developmental stage-dependency of the ABF4 effects implies that the growth retardation of *35S-ABF4* plants result probably from the constitutive operation of a part of ABA signal transduction cascades rather than from the pleiotropic effects of ABF4 overexpression. Except the varying degrees of growth retardation, neither *35S-ABF4* nor *35S-ABF3* plants did show abnormality in general development. Thus, their overexpression affected growth rate, but not developmental processes.

Other ABA- or stress-associated phenotypes of *35S-ABF3* and *35S-ABF4* transgenic plants include their hypersensitivities to salt and glucose, and *35S-ABF4* plants exhibited additional phenotypes (i.e., epinasty of leaves and abnormal obstacle-touching response) that can be related to altered ethylene or auxin response. The salt and glucose hypersensitivities may reflect the increased sensitivity to high osmolarity, since both high salt and high sugar accompany increase in osmolarity. However, *35S-ABF* plants responded normally to mannitol, indicating that osmotic sensitivity was not affected. Thus, it appears that ABF3 and ABF4 are involved only in the non-osmotic branches of salt and glucose signaling pathways. The sugar-mediated developmental arrest is confined to a narrow window of developmental stages, approximately 2 days postgermination, and is mediated by increased ABA level via an ABI4-dependent signaling cascade (Gazzarrini and McCourt, 2001). Also, it has been reported that ABA mediates developmental arrest at the similar stage and that the inhibition process requires ABI5 (Lopez-Molina et al., 2001), whose mutations result in weak sugar insensitivity. Thus, our results suggest that ABF3 and ABF4 have overlapping functions with ABI4 and ABI5 in mediating the sugar- and ABA-induced developmental arrest. This is particularly so in the case of ABF3, since the onset of its expression coincides with the early developmental stage (Figure 7B, a).

Stomatal closure is a key ABA-controlled process in coping with water deficit conditions. Our data indicate that ABF3 and ABF4 are involved in this process. Their overexpression resulted in lower transpiration and enhanced drought tolerance (Figure 5), which are reminiscent of the phenotypes of the ABA hypersensitive mutant *era1* (Pei et al., 1998). Furthermore, the stomatal openings of *35S-ABF* transgenic plants were smaller than those of wild type plants, and altered expression of several genes involved in stomatal aperture regulation has been observed in the transgenic plants (Figure 6). Among the genes we investigated, *ABI1* was the most strongly affected, especially in ABF3 overexpression lines. ABI1 is known to be a negative regulator of ABA signaling (Gosti et al., 1999), although its expression is enhanced by ABA and high osmolarity while reduced in the *aba1* and *abi1* mutants (Leung et al., 1997). It is not clear, thus, whether the increased *ABI1* level played a positive or a negative role in the stomatal closing. Whatever the ABI1's role might be, our results indicate that *ABI1* expression is subject to ABF3 regulation and that ABF3 and ABF4 overexpression affected the expression of genes involved in stomatal movement and/or guard cell ABA signaling (Schroeder et al., 2001), the net result being enhanced stomatal closure.

The transcript level changes of ABA responsive genes in *35S-ABF* transgenic lines demonstrate that ABF3 and ABF4 function as transcriptional regulators in planta. As shown in Figure 6, both positive and negative changes were observed depending on specific genes. The result suggests that different subsets of ABF3 and ABF4 target genes are regulated via different mechanisms. Also, the negative regulation of some genes under normal growth condition but positive regulation of the same genes after salt treatment (Figure 6, Group III) suggest that stress-induced modification of ABFs activities is required for the regulation of these genes. The modifying activity may be limiting under normal condition. Thus, the negative regulation of some genes can be explained, for example, by the binding of transcriptionally inactive, unmodified ABFs, whose proportion increases with higher ABF levels. The modification may involve phosphorylation of ABFs. Involvement of kinase/phosphatases in ABA/stress signalling has been well known (Leung and Giraudat, 1998), and several phosphorylation sites are highly conserved among ABFs (Choi et al., 2000). More recently, Uno et al. (Uno et al., 2000) reported an ABA-activated kinase activity in cultured cells that phosphorylates AREB1 (ABF2) and AREB2 (ABF4). Alternatively, it may involve interaction with other regulatory proteins.

The expression patterns of ABF3 and ABF4 were consistent with their functions suggested by their overexpression phenotypes. Spatially, both promoters were most active in roots and guard cells, consistent with their roles during water stress response. Also, ABF3, which exerted only minor growth inhibition, was weakly expressed in the growing tissues (root tips, new shoots, and new leaves), whereas ABF4 was strongly expressed in the growing regions of roots (meristem, elongation zone and lateral root primordia). Temporally, strong ABF3 promoter activity was observed in the newly germinated seedlings, consistent with its more pronounced effect on the germination (Figures 1C and 2C). On the other hand, the ABF4 promoter exhibited major activity at the onset of seedling growth, in agreement with its severe effect on seedling growth. Our results also show that both ABF3 and ABF4 might function during reproductive stages and seed abscission. Both promoters exhibited strong activity in the abscission zone, replum and funiculi of siliques, and relatively strong activity was also detected in stigma and anthers.

The isolation of multiple factors with similar binding activities but with different expression patterns suggested that the ABA/stress signaling involving the ABREs is likely to be mediated by multiple factors (Choi et al., 2000). Our current results further support the observation. Although their overexpression phenotypes and expression patterns were similar, ABF3 and ABF4 were different from each other in several respects. The details of the temporal and spatial expression patterns differed (Figure 7). Growth retardation, root growth inhibition and impaired stimulus-touching response were more prominent in ABF4 transgenic lines. Also, minor differences were observed at the molecular level. *ABI1* and *ABI2* expression levels were higher in *35S-ABF3* transgenic lines, while down-regulation of *SKOR* expression was observed only in *35S-ABF4* plants. The function of other ABFs, ABF1 and ABF2, remains to be determined, but their roles appear to be quite different from each other and also from ABF3 and ABF4 according to our preliminary data. Thus, ABRE-dependent ABA/stress signaling in vegetative tissues appears to be mediated by multiple factors with overlapping, but distinct functions.

Our data presented and discussed above indicate that ABF3 and ABF4 are involved in stress-responsive ABA signaling. In particular, overexpression of ABF3 and ABF4 clearly improved the survival rate of Arabidopsis plants under drought conditions. In our experiments, we used a constitutive promoter (i.e, 35S promoter). However, other promoters, which are tissue-specific or inducible, also can be employed to drive the expression of ABF genes in transgenic plants. Inducible promoters that are active only under stress conditions will be particularly effective in the case of ABF4, whose constitutive overexpression results in growth retardation. Also, it will be possible to alter stress tolerance of plants by decreasing the expression levels of ABFs or its homologs employing established technologies such as antisense, RNA interference and knockdown.

ABA controls not only the drought response but also responses to other environmental stresses such as high salt (Shinozaki and Yamaguchi-Shinozaki, 1997), cold/freezing (Llorente et al, 2000), heat (Larkindale J, Knight MR., 2002) and wounding (Giraudat et al, 1994). Thus, ABFs can be used to develop transgenic plants that are more tolerant than untransformed plants to these multiple stresses. Furthermore, the *cis*-regulatory elements which control stress-responsive gene expression and to which ABFs bind are highly conserved among various plants, in both monocot and dicot plants. Thus, overexpression of ABFs in plants other than Arabidopsis will also enhance their drought and other stress tolerance, and it will be possible to develop transgenic vegetable or cereal crop plants with enhanced stress tolerance by expressing ABF genes in them.

### EXAMPLES

### Arabidopsis Growth

*Arabidopsis thaliana* ecotype Landsberg *erecta* (L*er*) was used in this study. *aba1-1, abi1-1*, and *abi2-1* seeds (Koornneef et al., 1982, 1984) were obtained from the Arabidopsis Biological Resource Center at the Ohio State University, and their phenotypes were confirmed before use.

Plants were grown at 22 °C under long day condition (16 hr light/8 hr dark cycle) aseptically or on soil. For soil growth, seeds were sown on 1:1:1 mixture of vermiculite, perlite and peat moss irrigated with 0.1% Hyponex, placed at 4 °C for 4 days in the dark to break residual dormancy, and transferred to normal growth conditions. Unless otherwise stated, the plants were watered once a week. For aseptic growth, seeds were treated with 70% ethanol for 5 min and then with 30% household bleach for 5 min, washed 5 times with sterile water, and plated on MS medium (Murashige and Skoog, 1962) solidified with 0.8% Phytoagar. The MS medium was supplemented with 1% sucrose and, as described in the text, was also supplemented with ABA, salts, glucose, or mannitol as needed. For germination test, seeds collected at the same or similar times were used. For root growth measurement, plants were germinated and grown at vertical position.

### Constructs and Arabidopsis Transformation

The *35S* promoter-*ABF* coding region constructs (*35S-ABF3* and *35S-ABF4*) were prepared by replacing the β-glucuronidase (GUS) coding region of pBI121 (Jefferson et al., 1987) with the coding region of *ABF3* or *ABF4*. The *GUS* sequence was removed after BamHI-SacI digestion, and, after T4 DNA polymerase treatment to remove the 3'overhang, the remaining portion of pBI121 was ligated with the *ABF3* or the *ABF4* coding region, which was prepared by polymerase chain reaction (PCR) followed by BamHI digestion. The *ABF* coding regions included their entire coding regions with the stop codons and a BamHI linker sequence was attached in front of the initiation codons for the cloning purpose. The *ABF* promoter-*GUS* reporter fusions were prepared by inserting 2.1 kb (ABF3) or 1.2 kb (ABF4) of their 5' flanking sequences from the initiation codons in front of the GUS reporter gene of pBI101.2 (Jefferson et al., 1987). The promoter fragments were prepared by PCR using *Arabidopsis* (ecotype, Columbia) genomic DNA as a template and primer sets, 5'-caaacttaccctgttgttgcaact-3' (SEQ ID NO: 1) and 5'-ctagtctagaaggatcaagcttctggatatttac-3' (SEQ ID NO: 2) for *ABF3*, and 5'-gatcaatttgaatttttgatatacatc-3' (SEQ ID NO: 3) and 5'-ctagtctagattcaatgaaaacaaagcatccaag-3' (SEQ ID NO: 4) for *ABF4*. The PCR fragments were digested with XbaI and ligated with the pBI101.2, which was prepared by HindIII digestion followed by Klenow treatment and XbaI digestion. DNA manipulation was according to the standard procedures (Ausubel et al., 1994; Sambrook et al., 1989), and the intactness of the *ABF* coding regions and the junction sequences was confirmed by DNA sequencing.

Transformation of *Arabidopsis* was according to the vacuum infiltration method (Bechtold and Pelletier, 1998), using *A. tumefaciens* strain GV3101. For the phenotypic investigation, T3 or T4 homozygous lines were used. GUS staining patterns were confirmed by observing at least 5 different transgenic lines and T3 homozygous lines were used for detailed analysis.

### RNA Isolation, RT-PCR and RNA Gel Blot Analysis

RNA was isolated by the method of Chomczynski and Mackey (1995), with a minor modification (Choi et al., 2000). RNA gel blot analysis and RT-PCR were performed as described (Choi et al., 2000) with following modifications. For RNA gel blot analysis, hybridization was performed at 65 °C in the Rapid-hyb buffer from Amersham Pharmacia Biotech. Exposure time was 6 (ABF3) or 20 hr (ABF4). RT-PCR was performed using the Access RT-PCR System from Promega or the SUPERSCRIPT™ One-Step RT-PCR System from GIBCO BRL. Each RT-PCR result was confirmed by several independent reactions, and free of DNA contamination in RNA preparations was confirmed by using primer sets spanning introns whenever possible. Primers used in the RT-PCR reactions are presented in Table 1.

**Table 1.**

| **RT-PCR Primers** | |
|---|---|
| **Gene Name** | **Sequence (5' to 3')** |
| *actin* | F: cat cag gaa gga ctt gta cgg |
| | R: gat gga cct gac tcg tca tac |
| *ABF3* | F: aga acc tca acc ggt gga gag tg |
| | R: gga gtc aga tca ggt gac atc tgg |
| *ABF4* | F: aac tgt gtt caa cag atg ggt cag |
| | R: ggt tec tcc gta act agc taa tcc |
| *rd29B* | F: gtg aag atg act atc tcg gtg gtc |
| | R: gcc taa ctc tcc ggt gta acc tag |
| *rab18* | F: atg acg agt acg gaa atc cga tgg |
| | R: tat gta tac acg att gtt cga agc |
| *ICK1* | F: acg cac acg taa cct aaa tcg |
| | R: gca tct ccg tca tca att tcg |
| *ABI1* | F: tca aga ttc cga gaa cgg aga tc |
| | R: gag gat caa acc gac cat cta ac |
| *ABI2* | F: gtt ctt gtt ctg gcg acg gag c |
| | R: cca tta gtg act cga cca tca ag |
| *rd29A* | F: gat aac gtt gga gga aga gtc ggc |
| | R: cag ctc agc tcc tga ttc act acc |
| *SKOR* | F: atg gga ggt agt agc ggc ggc |
| | R: gat tct ctg gta atc ccc tga ag |
| *KAT1* | F: ttc tgc gtc gag gaa tac aat ata g |
| | R: ctt agg gtc aac tag aag ata g |
| *KAT2* | F: aca caa gac caa tgt caa tct ctt g |
| | R: gtc gac tag aag ata tga gtg gc |
| *ADH1* | F: tcc acg tat ctt cgg cca tg |
| | R: tag cac ctt ctg cag cgc c |
| *CHS* | F: tca cca aca gtg aac aca tga cc |
| | R: gag tca agg tgg gtg tca gag g |
| F: forward primer, R: reverse primer | |

### Histochemical GUS staining

In situ assay of GUS activity was performed as described by Jefferson et al. (1987). Whole plants were immersed in 1 mM X-gluc (5-bromo-4-chloro-3-indolyl-β-glucuronic acid) solution in 100 mM sodium phosphate, pH 7.0, 0.1 mM EDTA, 0.5 mM ferricyanide, 0.5 mM ferrocyanide, and 0.1% Triton X-100, and after applying vacuum for 5 min, incubated at 37 °C for indicated times. Chlorophyll was cleared from the plant tissues by immersing them in 70% ethanol.

### Drought Treatment and Measurement of Transpiration Rate

For drought treatment, 3 week-old, soil-grown plants were withheld completely from water for the specified times. To minimize experimental variations, same numbers of plants were grown on the same tray. With *35S-ABF4* plants, which show growth retardation, two batches of plants, one of the same age and the other of similar developmental stages (i.e., wild type seeds were sown 7 days later so that they were at the similar developmental stages at the end of the treatment) were tested, and similar results were obtained. The whole test was repeated at least four times, sometimes using different arrangements of plants (i.e., test plants on different containers etc.), and the results were consistent. Transpiration rate of detached leaves was measured by weighing freshly harvested leaves placed abaxial side up on open petri dishes on the laboratory bench. Leaves of similar developmental stages (third to fifth true rosette leaves) from three week-old, soil-grown plants were used.

### Guard Cells

To examine guard cells, leaves were excised from 3 week-old, soil-grown plants in the middle of watering (3 days after watering) and light periods. Leaves of similar developmental stages (third to sixth true rosette leaves) from 20 different plants of wild type and transgenic lines, respectively, were placed on slides abaxial side up immediately after excision and pictures were taken. The number of guard cells was then counted in the randomly chosen fields, usually 6-7.

### REFERENCES

**Anderson, J.A., Huprikar, S.S., Kochian, L.V., Lucas, W.J., and Gaber, R.F.** (1992). Functional expression of a probable *Arabidopsis thaliana* potassium channel in *Saccharomyces cerevisiae*. Proc. Natl. Acad. Sci. U.S.A. **89,** 3736-3740.

**Arenas-Huertero, F., Arroyo, A., Zhou, L., Sheen, J., and Leon, P.** (2000). Analysis of *Arabidopsis* glucose insensitive mutants, gin5 and gin6, reveals a central role of the plant hormone ABA in the regulation of plant vegetative development by sugar. Genes Dev. **14,** 2085-2096.

**Ausubel, F.M., Brent, R., Kingston, R.E., Moore, D.D., Seidman, J.G., Smith, J.A., and Struhl, K.** (1994). Current Protocols in Molecular Biology, New York, Green Publishing Associates/Wiley Interscience.

**Bartholomew, D.M., Bartley, G.E., and Scolnik, P.A.** (1991). Abscisic acid control of *rbcS* ad *cab* transcription in tomato leaves. Plant Physiol. **96,** 291-2926.

**Beaudoin, N., Serizet, C., Gosti, F., and Giraudat, J.** (2000). Interactions between abscisic acid and ethylene signaling cascades. Plant Cell **12,** 1103-16.

**Bechtold, N., and Pelletier, G.** (1998). *In planta Agrobacterium-mediated* transformation of adult *Arabidopsis thaliana* plants by vacuum infiltration. Methods Mol. Biol. **82,** 259-266.

**de Bruxelles, G.L., Peacock, W.J., Dennis, E.S., and Dolferus, R.** (1996). Abscisic acid induces the alcohol dehydrogenase gene in *Arabidopsis.* Plant Physiol. **111**, 381-391.

**Busk, P.K., and Pages, M.** (1998). Regulation of abscisic acid-induced transcription. Plant Mol. Biol. 37, 425-435.

**Choi, H., Hong, J., H, J., Kang, J., and Kim, S.Y.** (2000). ABFs, a family of ABA-responsive element binding factors. J. Biol. Chem. **21,** 1723-1730.

**Chomczynski, P., and Mackey, K.** (1995). Modification of the TRI reagent procedure for isolation of RNA from polysaccharide- and proteoglycan-rich sources. *BioTechniques* **19,** 942-945.

**Feinbaum, R.L., and Ausubel, F.M.** (1988). Transcriptional regulation of the *Arabidopsis thaliana* chalcone synthase gene. Mol Cell Biol **8,** 1985-1992.

**Finkelstein, R.R., and Lynch, T.J.** (2000). The *Arabidopsis* abscisic acid response gene *ABI5* encodes a basic leucine zipper transcription factor. Plant Cell **12,** 599-609.

**Finkelstein, R.R., Wang, M.L., Lynch, T.J., Rao, S., and Goodman, H.M.** (1998). The *Arabidopsis* abscisic acid response locus *ABI4* encodes an APETALA 2 domain protein. Plant Cell **10,** 1043-54.

**Foster, R., Izawa, T., and Chua, N.-H.** (1994). Plant bZIP proteins gather at ACGT elements. FASEB J. **8,** 192-200.

**Gaymard, F., Pilot, G., Lacombe, B., Bouchez, D., Bruneau, D., Boucherez, J., Michaux Ferriere, N., Thibaud, J.B., and Sentenac, H.** (1998). Identification and disruption of a plant shaker-like outward channel involved in K⁺ release into the xylem sap. Cell **94,** 647-55.

**Gazzarrini, S., and McCourt, P.** (2001). Genetic interactions between ABA, ethylene and sugar signaling pathways. Curr. Opin. Plant Biol. **4,** 387-391.

**Ghassemian, M., Nambara, E., Cutler, S., Kawaide, H., Kamiya, Y., and McCourt, P.** (2000). Regulation of abscisic acid signaling by the ethylene response pathway in *Arabidopsis*. Plant Cell. **12,** 1117-1126.

**Giraudat, J., Hauge, B.M., Valon, C., Smalle, J., Parcy, F., and Goodman, H.M.** (1992). Isolation of the *Arabidopsis ABI3* gene by positional cloning. Plant Cell. **4,** 1251-61.

**Giraudat, J., Parcy, F., Bertauche, N., Gosti, F., and Leung, J.** (1994). Current advances in abscisic acid action and signaling. Plant Mol. Biol. **26**, 1557-1577.

**Giuliano, G, Pichersky, E., Malik, V.S., Timko, M.P., Scolnik, P.A., and Cashmore, A.R.** (1988). An evolutionary conserved protein binding sequence upstream of a plant light-regulated gene. Proc. Natl. Acad. Sci. USA **85,** 7089-7093.

**Gosti, F, Beaudoin, N., Serizet,C., Webb. A.A., Vartanian, N., and Giraudat, J.** (1999). ABI1 protein phosphatase 2C is a negative regulator of abscisic acid signaling. Plant Cell. **11**, 1897-910.

**Himmelbach, A., Iten, M., and Grill, E.** (1998). Signalling of abscisic acid to regulate plant growth. Phil. Trans. R. Soc. London. B **353**, 1439-1444.

**Huijser, C., Kortstee, A., Pego, J., Weisbeek, P., Wisman, E., and Smeekens, S.** (2000). The *Arabidopsis SUCROSE UNCOUPLED-6* gene is identical to *ABSCISIC ACID INSENSITIVE-4:* involvement of abscisic acid in sugar responses. Plant J. **23,** 577-585.

**Ingram, J., and Bartels, D.** (1996). The molecular basis of dehydration tolerance in plants. Ann. Rev. Plant Physiol. Plant Mol. Biol. **47**, 377-403.

**Jang, J.-C., Leon, P., Zhou, L., and Sheen, J.** (1997). Hexokinase as a sugar sensor in higher plants. Plant Cell **9,** 5-19.

**Jackson, M.** (1997). Hormones from roots as signals for the roots of stressed plants. Trends Plant Sci. **2,** 22-28.

**Jefferson, R.A., Kavanagh, T.A., and Bevan, M.W.** (1987). GUS fusions: β-glucuronidase as a sensitive and versatile gene fusion marker in higher plants. EMBO J. **20**, 3901-3907.

**Koornneef, M., Jorna, M.L., Brinkhorst-van-der Swan, D.L.C., and Karssen, C.M.** (1982). The isolation of Abscisic Acid (ABA) deficient mutants by selection of induced revertants in non-germinating gibberellin sensitive lines of *Arabidopsis thaliana* (L.) Heynh. Theor. Appl. Genet **61**, 385-393.

**Koornneef, M., Reuling, G., and Karssen, C.M.** (1984). The isolation and characterization of abscisic acid-insensitive mutants of *Arabidopsis thaliana*. Phsiol. Plant. **61,** 377-383.

**Laby, R.J., Kincaid, M.S., Kim, D., and Gibson, S.I.** (2000). The *Arabidopsis* sugar-insensitive mutants *sis4* and *sis5* are defective in abscisic acid synthesis and response. Plant J. **23,** 587-596.

**Landschulz, W.H., Johnson, P.F., and McKnight, S.L.** (1988). The leucine zipper: A hypothetical structure common to a new class of DNA binding proteins. Science **240,** 1759-1764.

**Lang, V., and Palva, E.T.** (1992). The expression of a *rab*-related gene, *rab18*, is induced by abscisic acid during the cold acclimation process of *Arabidopsis thaliana* (L.) Heynh. Plant Mol. Biol. **20**, 951-962.

**Larkindale J, Knight MR.** (2002). Protection against heat stress-induced oxidative damage in Arabidopsis involves calcium, abscisic acid, ethylene, and ssalicylic acid. Plant Physiol. 128, 682-695.

**Leon-Kloosterziel, K.M., Gil, M.A., Ruijs, G.J., Jacobsen, S.E., Olszewski, N.E., Schwartz, S.H., Zeevaart, J.A., and Koornneef, M.** (1996). Isolation and characterization of abscisic acid-deficient *Arabidopsis* mutants at two new loci. Plant J. **10**, 655-661.

**Leung, J., Bouvier-Durand, M., Morris, P.C., Guerrier, D., Chefdor, F., and Giraudat, J.** (1994). *Arabidopsis* ABA response gene ABI1: features of a calcium-modulated protein phosphatase. Science **264,** 1448-52.

**Leung, J., and Giraudat, J.** (1998). Abscisic acid signal transduction. Ann. Rev. Plant Physiol. Plant Mol. Biol. **49**, 199-222.

**Leung, J., Merlot, S., and Giraudat, J.** (1997). *Arabidopsis* ABA response gene ABI1: features of a calcium-modulated protein phosphatase. *Plant Cell***9**, 759-71.

**Llorente, F., Oliveros, J.C., Martinez-Zapater, J.M. & Salinas, J.** (2000). A freezing-sensitive mutant of *Arabidopsis*, *frs1*, is a new *aba3* allele. *Planta* **211,** 648-655.

**Lopez-Molina, L., and Chua, N.-H.** (2000). A null mutation in a bZIP factor confers ABA-insensitivity in *Arabidopsis thaliana*. Plant Cell Physiol. **41,** 541-547.

**Lopez-Molina, L., Mongrand, S., and Chua, N.-H.** (2001). A postgermination developmental arrest checkpoint is mediated by abscisic acid and requires the ABI5 transcription factor in *Arabidopsis*. Proc. Natl. Acad. Sci. U. S. A. **98,** 4782-4787.

**Menkens, A.E., Schindler U., and Cashmore, A.R.** (1995). The G-box: a ubiquitous regulatory DNA element in plants bound by the GBF family of bZIP proteins. Trend Biochem Sci. **20,** 506-512.

**Meyer, K., Leube, M.P., and Grill E.** (1994). A protein phosphatase 2C involved in ABA signal transduction in *Arabidopsis thaliana*. Science **264,** 1452-5.

**Murashige, T., and Skoog, F.** (1962). A revised medium for rapid growth and bioassay with tobacco tissue culture. Physiol. Plant. **15,** 473-497.

**Okada, K., and Shimura, Y.** (1990). Reversible root tip rotation in *Arabidopsis* seedlings induced by obstacle-touching stimulus. Science **250,** 274-276.

**Okada, K., and Shimura, Y.** (1992). Aspects of recent development in mutational studies of plant signaling pathways. Cell **70**, 369-372.

**Pei, Z.M., Ghassemian, M., Kwak, C.M., McCourt, P., and Schroeder, J.I.** (1998). Role of farnesyltransferase in ABA regulation of guard cell anion channels and plant water loss. Science. **282**, 287-290.

**Pilot, G., Lacombe, B., Gaymard, F., Cherel, I., Boucherez, J., Thibaud,J.B., and Sentenac,H.** (2001). Guard cell inward K+ channel activity in *Arabidopsis* involves expression of the twin channel subunits KAT1 and KAT2. J. Biol. Chem. **276**, 3215-3221.

**Quesada, V., Ponce, M.R., and Micol, J.L.** (2000). Genetic analysis of salt-tolerant mutants in *Arabidopsis thaliana. Genetics* **154**, 421-436.

**Sambrook, J., Fritsch, E.F., and Maniatis, T.** (1989) Molecular Cloning: A laboratory Manual, 2^{nd} ed., Cold Spring Harbor Laboratory, Cold Spring Harbor, NY.

**Schroeder, J.I., Kwak, J.M., and Allen, G.J.** (2001). Guard cell abscisic acid signaling and engineering drought hardiness in plants. Nature **410**, 327-330.

**Shinozaki, K., and Yamaguchi-Shinozaki, K.** (1997). Gene expression and signal transduction in water-stress response. Plant Physiol. **115,** 327-334.

**Simmons, C., Soll, D., and Migliaccio, F.** (1995). Circumnutation and gravitropism cause root waving in *Arabidopsis thaliana. J.* Exp. Bot. **46**, 143-150.

**Uno, Y., Furihata, T., Abe, H., Yoshida, R., Shinozaki, K., and Yamaguchi-Shinozaki, K.** (2000). *Arabidopsis* basic leucine zipper transcription factors involved in an abscisic acid-dependent signal transduction pathway under drought and high-salinity. Proc. Natl. Acad. Sci. U. S. A. **97**, 11632-7.

**Vartanian, N., Marcotte, L., and Giraudat, J.** (1994). Drought rhizogenesis in *Arabidopsis thaliana*. Plant Physiol. **104**, 761-767.

**Wang, H., Qi, Q., Schorr, P., Cutler, A.J., Crosby, W.L., and Fowke, L.C.** (1998). ICK1, a cyclin-dependent protein kinase inhibitor from *Arabidopsis thaliana* interacts with both Cdc2a and CycD3, and its expression is induced by abscisic acid. Plant J. **15**, 501-510.

**Wang, S.M.., Lue, W.L., Eimert, K., and Chen, J.** (1996). Phytohormone-regulated beta-amylase gene expression in rice. Plant Mol. Biol. **31**, 975-982.

**Weatherwax, S.C., Ong, M.S., Degenhardt, J., Bray, E.A., and Tobin, E.M.** (1996). The interaction of light and abscisic acid in the regulation of plant gene expression. Plant Physiol **111,** 363-370.

**Werner, J.E., and Finkelstein, R.R.** (1995). *Arabidopsis* mutants with reduced response to NaCl and osmotic stress. Physiol. Plant. **93,** 659-666.

**Yamaguchi-Shinozaki, K., and Shinozaki, K.** (1994). A novel cis-acting element in an *Arabidopsis* gene is involved responsiveness to drought, low-temperature, or high-salinity stress. Plant Cell 6, 251-264.

**Zeevaart, J.A.D., and Creelman, R.A.** (1988). Metabolism and physiology of abscisic acid. Ann. Rev. Plant Physiol. Plant Mol. Biol. **39,** 439-73.

**Zhu, J.-K.** (2000). Genetic Analysis of Plant Salt Tolerance Using Arabidopsis. Plant Physiol. **124,** 941-948.

## Claims

1. Transgenic plants, the cells of which comprise a recombinant DNA segment encoding an abscisic acid responsive element binding factor ABF, wherein expression of the factor is altered to confer enhanced tolerance to reduced water availability and other environmental stresses to the transformed plants.

2. The transgenic plant according to claim 1, wherein said abscisic acid responsive element binding factor is ABF3 and/or ABF4.

3. A seed produced by the transgenic plants of claim 1.

4. A progeny, clone, cell line or cell of the transgenic plants of claim 1.

5. A method to increase plants' tolerance to reduced water availability and other environmental stresses, comprising the steps of:
i ) constructing an expression vector comprising a regulatory sequence for over- or under-expression of ABFs and an ABF coding sequence in the sense or antisence orientation;
ii) transferring the expression vector to plant cells by *Agrobacterium*-mediated transformation, by biolistic transformation or by other established plant transformation methods,
iii) selecting and maintaining the transgenic individuals expressing the recombinant ABF.
